Europäisches Patentamt

(19) European Patent Office

Office européen des brevets

(11) Publication number: **0 166 051**
**B1**

# (12) EUROPEAN PATENT SPECIFICATION

(45) Date of publication of patent specification: **15.03.89**

(21) Application number: **84304417.3**

(22) Date of filing: **28.06.84**

(51) Int. Cl.⁴: **A 61 F 2/16, B 29 D 11/02**

(54) Process for fabricating an intraocular lens.

(43) Date of publication of application:
**02.01.86 Bulletin 86/01**

(45) Publication of the grant of the patent:
**15.03.89 Bulletin 89/11**

(84) Designated Contracting States:
**DE FR GB IT**

(56) References cited:
**CH-A- 411 323**
**GB-A-2 114 315**
**US-A-3 866 249**
**US-A-3 913 148**
**US-A-3 996 627**
**US-A-4 073 015**
**US-A-4 253 199**
**US-A-4 254 509**
**US-A-4 373 218**
**US-A-4 449 257**

(73) Proprietor: **Ceskoslovenska akademie ved**
**Narodni trida 3**
**Praha 1 (CS)**

(72) Inventor: **Eichterle, Otto**
**27 Na Andelce**
**Praha 6 (CS)**

(74) Representative: **Matthews, Howard Nicholas**
**BROOKES & MARTIN Incorporating**
**MATTHEWS, HADDAN & CO. High Holborn**
**House 52/54, High Holborn**
**London, WC1V 6SE (GB)**

Courier Press, Leamington Spa, England.

**Description**

Background of the invention
Field of the invention

This invention relates to intraocular lenses of hydrogel material adapted for implantation in a human eye.

Discussion of the prior art

The concept of the use of intraocular lenses for the correction of aphakia has a long history. Much of the pioneering work was performed by Harold Ridley in London and by Binkhorst in the Netherlands. A comprehensive history of the development and results of the intraocular implant lens is presented by a thesis by Marcel Eugene Nordlohne and reproduced in Documenta Ophthalmologica, Vol. 38, Issue 1, Dec. 16, 1974.

A summary of the history of various artificial lens implants is also found in an article written by D. P. Choyce, published in the Annals of Ophthalmology, October, 1973, pages 1113—1120. In most cases lenses were made from organic high polymers such as, for example, poly(methyl methacrylate). It has also been proposed to implant glass lenses and to utilize lenses made of pure silicate glass. Specific glass compositions have been proposed in the disclosure of U.S. Pat. No. 3,996,627, for example. Although glasses have certain advantages as ocular implants, they are, in most cases, of a density rendering finished lenses relatively high in weight and less than optimum in this respect as implants. An early report by Schiferli in 1795 described an attempt by Casaamata to introduce a glass lens into the eye after a cataract operation. The lens immediately slipped back toward the fundus of the eye.

Many patents are addressed to the problem of mounting the intraocular lens within the eye so that it can perform its intended function with a minimum of trauma to the eye; see for example U.S. Pat. Nos. 2,834,023; 3,711,870; 3,673,616; 3,866,249; and 3,913,148.

Intracameral lenses, i.e., prosthesis for the removed natural lens, fabricated of poly(methyl methacrylate) or of many glass-like materials generally are characterized by a refractive index value which is substantially greater than the refractive index value of 1.396 of the natural crystalline lens of a human eye. For example, intraocular-lenses of the poly(methyl methacrylate) type possess a refractive index in the area of 1.53. Thus, the geometry and shape of an intraocular implant lens of the poly(methyl methacrylate) type differ from that of a natural lens to enable the artificial lens immersed in the refracting medium (the aqueous humor and vitreous humor) to substantially duplicate the refractive index characteristic of the natural lens. For example, the artificial lens is shaped with substantially smaller curvatures of both optical surfaces than is the case with the natural lens. However, such changes in shape of the artificial lens precludes implantation in the exact position of the space occupied by the natural lens. Further disparity is noted in the specific weights of conventional material, e.g., about 1.25, used in the fabrication of intraocular lenses compared with the specific weight of the natural lens (about 1.1). Thus an IOL implant of conventional material immersed in the refracting medium of the eye appears several times heavier than the natural lens. This disadvantage has been diminished by the recent practice of designing thin intracameral lenses which, however, further increases the diversity of shape between the artificial lens and the natural lens.

Many adaptations have been proposed, and several actually used, to hold a miniaturized but relatively heavy intraocular lens system (body and mounting or securing means) in the desired optical zone. Pat. No. 4,073,015 discloses the mounting and securing of an intraocular lens so as to hold it in place permanently without lasting irritation to the patient. The patentees state that the lens is made of acrylic, hydrogel, or other biologically tolerable lens material and is formed with laterally extending planar flanges or haptical portions. A woven or knitted fibrous material, such as Dacron, is attached to the outer perimeter of the haptical portions and provide sites into which tissue of the iris can grow so as to form a permanent anchor for the lens; see column 1, line 66 to column 2, line 7 of U.S. 4,073,015. The intraocular lens structure and lens mounting system (lens body, integral haptical portion, loops, etc.) is inserted into the location previously occupied by the crystalline lens by expending the pupillary opening of the iris to admit the said system; column 3, lines 56—66 of U.S. 4,073,015. It is apparent from the specification and drawings that the lens body of the patentees' intraocular lens systems is significantly smaller than the natural lens of the patient's eye.

One form of intraocular lens is described in U.S. Patent 3866249 (Flom). Another such lens is described in U.S. Patent 4449257 (Koeniger). This being cut from hard HEMA plastic. U.S. Patents 4449257 and 3866249 disclose in artificial intraocular lens adapted to be implanted in the posterior chamber of a human eye which consists of an intraocular lens body having a shape substantially defined by two rotationally symmetrical and coaxial optically finished surfaces, at least one surface being convex. Said lens consisting essentially of water swellable, water insoluble stable hydrogel material. CH—A—411323 (Ceskoslovenska Akademie Ved) describes a contact lens made from hydrophilic polymerizable liquid material introduced in liquid condition into a concave rotating mould and polymerized therein, the liquid being a solution or mixture suitable for three dimensional polymerization, and being an olefinic unsaturated hydrophilic monomer so that the outer surface of the lens through the rigid mould and the inner surface through the rotation of the mould and the upper surface tension are formed.

## Summary of the invention

According to the invention we provide a process for fabricating an intraocular lens of predetermined dimensions from a polymerizable hydrogel liquid mixture comprising reactants, characterized by:

a. introducing the liquid mixture into a cavity of an open mold which has a concave surface corresponding to the convex shape of one surface of the predetermined intraocular lens, said cavity perimeter being delimited by a sharp edge;

b. said liquid mixture being introduced in an amount at least sufficeint to approximately reach the level of the sharp edge such that the amount may exceed the volume of the cavity defined by the sharp edge, may be level with the sharp edge or may be slightly below the sharp edge in order to form respectively a convex, plano or slightly concave front surface which is not in contact with the mold;

c. polymerizing the liquid mixture without rotating the mold, for a period of time sufficient to form a hydrogel intraocular lens having at least one convex surface;

d. equilibrating said intraocular lens product by repeating washings in an aqueous medium; and

e. thereafter storing and maintaining the resulting predetermined intraocular lens, at least one optically finished convex surface of the lens being in osmotic equilibrium with a physiological solution.

## Brief description of the drawings

Figure 1 is a cross-sectional side view of concave mold 10 with curves 11, 12, 13 and 14 representing side sections through the surface of convex menisci formed by overdosing mold cavity 4 with liquid comprising polymerizable reactants.

Figure 2 is a cross-sectional side view of the intraocular hydrogel lens as separated from mold 10. Curves 1 and 2 of this view of the IOL are in agreement with the curves defined by mold cavity side section 7 and convex maniscus side section 11 (Figure 1).

## Detailed description of the invention

This invention pertains to process for fabricating a stable intraocular lens body of hydrogel material useful as implants for the natural crystalline lens of a human eye removed by surgical means or otherwise. The intraocular hydrogel lens body has a shape substantially defined by two rotationally symmetrical and coaxial optically finished surfaces, at least one surface being convex, e.g., double-convex (preferred), plano-convex, and slightly concavo-convex, provided the required focal length is obtained. In a preferred form both surfaces, e.g., double-convex, of the lens body have a continuous transition, i.e., without sharp edges or sharp line of demarcation. The intraocular lens consists essentially of a water-swellable, water-insoluble, stable hydrogel which contains at least about 60% by weight, preferably at least about 65—70% by weight of water or physiologic saline (about 0.9% by weight saline solution) based on the total weight of the hydrogel. The hydrogel, in osmotic equilibrium with physiologic saline, possesses an index of refraction of about 1.4, desirably from about 1.37 to about 1.45, preferably from about 1.38 to about 1.42, and preferably still from about 1.39 to about 1.41. The upper limit of water content in the hydrogel is controlled by its ability to be generally shape-retaining with sufficient mechanical strength to function as an artificial lens body replacement for the opacified natural lens. In general, an upper water content limit of 90—95% by weight is suitable; 85—90% by weight is desirable in various embodiments. The hydrogels are made via polymerization processes and in this respect, the concentration and starting material(s) of choice, e.g., monomer(s), will significantly influence the water-swellability characteristic of the resulting polymer(s).

The hydrogel material, in the shape of the intraocular implant lens, has the following properties: transparency, good optics (giving it capability to function as an IOL), biologically inert, biocompatibility with living tissue, stability in normal physiologic medium, good mechanical properties such as softness, elasticity and modulus. The hydrogel material can be derived from synthetic or natural stable material, or modifications of both types, e.g., the various types of modified collagens and other natural products, polymerization products from ethylenically unsaturated polymerizable monomers, products from the condensation reaction of polyisocyanates and polyols (natural and synthetic), and the like. In a preferred form the hydrogal is a synthetically derived material.

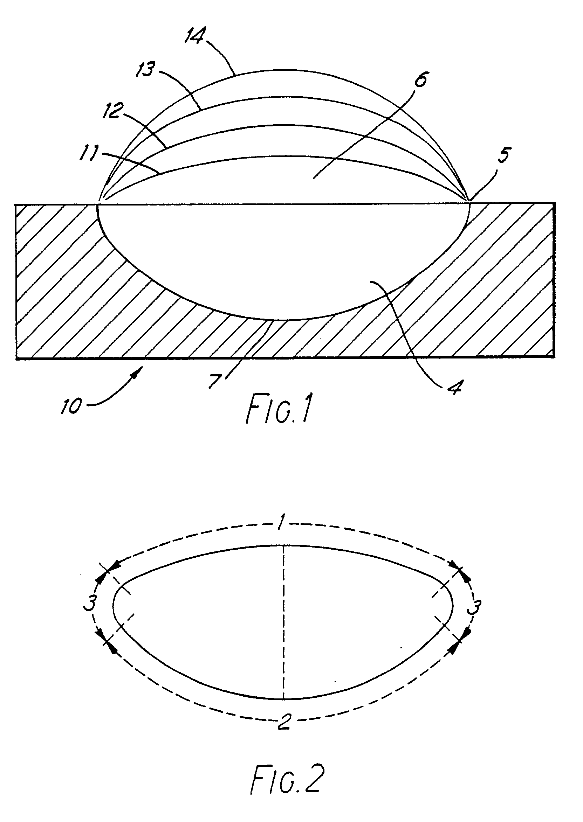

The general shape of the intracameral hydrogel lens approximates the general shape of the natural crystalline lens in the capsula lentis. Referring to Figure 2, the artificial lens (preferred) is characterized by a front surface 1 whose general shape resembles a flat ellipsoid having a central radius of curvature in the range of from about 7.5 to about 15 mm; a back surface 2 which is a spherical surface or a rotationally symmetrical surface of the second order, the central radius of curvature being in the range of from about 5 to 8 mm; two toroidal surfaces 3 delineated by front surface 1 and back surface 2 and having the shape of a common torus; and a central lens thickness (between front surface 1 and back surface 2) of from about 2.5 to about 5 mm.

Suitable intraocular lenses are fabricated from starting materials which are employed in the manufacture of so-called soft hydrophilic contact lens of high water content and other articles of similar hydrogel materials. Desirably the hydrogel is characterized by a relatively slightly to moderately crosslinked polymeric network and such products are well described in the literature. The operative conditions necessary to effect the appropriate reaction, especially the polymerization reaction, are of course well known in the art; see by way of illustrations U.S. Pat. Nos. 4,032,599; 4,067,839; 4,036,814; 4,095,877; 4,275,183; 4,361,689; 4,388,436; and 4,408,023.

One of ordinary skill in the art can select the monomer or mixture of monomers of choice, including the appropriate crosslinking means, catalyst(s), solvent, and the like. Illustrative reactants include hydrophilic monomers such as 2 - hydroxyethyl methacrylate, glycidyl methacrylate, N - vinylpyrrolidone, methacrylamide, acrylamide, N - methylacrylamide, methacrylonitrile, diethylene glycol monomethacrylate, alkali metal salts of itaconic acid, of methacrylic acid, and the like. The hydrophilicity of the polymeric product can oftentimes be enhanced by using, for example, methacrylic acid as one of the monomers.

Crosslinking means include, for example, di- or poly-functional species such as divinylbenzene, ethylene glycol diacrylate or dimethacrylate, propylene glycol diacrylate or dimethacrylate, and the polyacrylate or polymethacrylate esters of the following polyols: triethanolamine, glycerol, diethylene glycol, triethylene glycol, tetraethylene glycol, trimethylolpropane, and the like. Other crosslinking monomers can be illustrated by N,N - methylene - bis - acrylamide or methacrylamide and sulfonated divinylbenzene. Polymerization can also be effected using, for example, radiation (U.V., X-Ray, microwave, or other well-known forms of radiation) with/without the presence or well-known initiator(s) and/or catalyst(s) such as the organic peroxides, the·alkyl percarbonates, hydrogen peroxides, and inorganic materials such as ammonium, sodium, or potassium persulfate. Polymerization temperatures can vary from about 20°C, and lower, to about 100°C, and higher. Desirably the polymerization of the ethylenically unsaturated compounds is carried out in the presence of a liquid medium and/or plasticizer and/or solvent, e.g., water, glycerol, miscible organic liquid/water media, and the like.

Mixtures of reactants are generally preferred since hydrogels having "tailor-made" characteristics can be prepared. Mixtures of hydrophilic and hydrophobic reactants in appropriate concentrations can be utilized providing the resulting polymeric product has the capability of meeting the desired water content level illustrated previously. Illustrative hydrophobic monomers include the lower alkyl methacrylates such as methyl methacrylate.

The foregoing discussion is well documented in the literature, especially the body of patent literature relating to so-called soft contact lenses.

The liquid mixture for the lens comprises lens-forming reactant(s) and other ingredients, e.g., water (preferred) and/or miscible organic liquid/water (preferred) or organic medium such as glycerol (preferred) desirably into an open concave mold. The mold is fabricated from any suitable material which forms with the said mixture a wetting angle greater than zero. The concave mold cavity has the shape of a sphere or rotational symmetrical surface of second order and terminates with a horizontal circular sharp edge. The liquid mixture of monomer(s) is dosed into the mold cavity in a predetermined amount which generally exceeds (preferred) the volume of the cavity defined by the circular sharp edge. The liquid mixture does not flow over the circular edge, but forms a convex surface of the type of a flat rotational ellipsoid above the circular edge. In this manner the desired surfaces of the intraocular lens are formed in situ. In this procedure the wetting angle, as indicated above, is greater than zero. In other words, if the surface is unwettable (e.g., Teflon®) or is not completely wetted, the liquid mixture in excess of the volume of the mold cavity does not spill over, but rather it forms the convex menisci side sections shown as 11, 12, 13 and 14 in Figure 1. The larger the wetting angle the greater the amount of overdosing that can be tolerated. Of course, a point is reached where surface tension, adhesive forces, and other forces and attractions cannot hold the volume of liquid mixture within the bulging convex surface above the circular mold edge. A most suitable shape of the projecting meniscus of liquid mixture is achieved with dosed volumes (of liquid) which are about 10 to about 80 volume percent greater than the volume of the mold cavity *per se* (below sharp horizontal edge 5). An arbitrary central radius therefore can be obtained by adjustment of the amount of liquid overdosed above the volume of the mold cavity. One embodiment of the invention, as indicated previously, includes an IOL which can have a plano and even a slightly concave front surface. In such instances the dosing of mold cavity 4 with liquid mixture may slightly exceed sharp circular mold edge 5, may be level with mold edge 5, or may even be slightly below mold edge 5, depending on the IOL optics required by the patient. The liquid mixture comprising monomer(s) after dosing is subjected to polymerization conditions, e.g., polymerization temperature in an inert atmosphere or it is exposed to a photochemically effective light, if photoinitiators are used. On completion of the polymerization reaction, the molded product may be removed from the mold or the mold and product can be immersed in water where it (IOL product) is readily released from the mold after swelling. The IOL product does not require any additional mechanical treatment because its surface is optically precise and completely smooth. It needs only removal of extractable low-molecular weight impurities by required washings with water or aqueous alcohol and the final equilibration with physiological saline. Thereafter it can be sterilized and stored until used for the intended purpose.

In view of the soft and elastic shape retaining nature of the intraocular lens, the lens can be conveniently sterilized and stored, preferably in a tubular container, and thus made ready for use during primary (preferred) or secondary implantation operations. In one form the tubular container (e.g., fabricated of flexible plastic such as polyethylene) has a diameter at one end thereof which is smaller than the diameter (thickness) of the intraocular lens. The IOL can be forced to the narrower end of a flexible, thin walled container by exerting pressure on the container to cause the IOL to become sufficiently deformed, oblong-wise, so as to be capable of exiting from the narrow opening of the container. Pressure on the container can be conveniently effected by hand pressing the container (e.g., by the ophthalmologist) to the extent necessary to cause the deformation but not the rupture or loss of integrity of the intraocular lens. In amoeba-like fashion the IOL is thus squeezed out from its container and will thereafter readily assume its original size and shape. If the internal wall of a container, conveniently a flexible container such as plastic, e.g., polyethylene or polypropylene, is hydrophilized as by techniques known in the art, the movement of the IOL therein and its exit therefrom can be made quite facile. The intraocular lens may be fixed within the container in a manner whereby the front and back surfaces of the IOL are indicated on the container wall.

The IOL body can be inserted into the empty space created, for example, by the surgical extraction of a turbid lens. If the rear capsula lentis has not been removed it can function as a thin natural membrane or foil which as a rule is clear and which precisely delimits the space for an artificial lens of similar size and shape. The most closely fitting intraocular lens can be generally ascertained according to the shape and volume or weight of the removed natural lens. In this respect the ophthalmologist can choose from a large stock of the intracameral lenses and insert the appropriate lens into the space occupied by the removed lens. If the capsula lentis is intact after the removal of the turbid lens and if the ciliary muscles proximal thereto have not degenerated, the IOL, being sufficiently soft and elastic and occupying the space formerly occupied by the natural lens, can be at least partially deformed by the muscles (as in the functioning of a normal natural lens) thereby imparting at least partial accommodation to the eye of the patient.

The hydrated IOL can be introduced into the posterior chamber of the eye (utilizing a tubular container of the principle discussed previously) as through the pupillary aperture which is smaller than the diameter of the IOL in its normal relaxed state or through any small acceptable surgical opening to the posterior chamber. In view of the capability of the intraocular lens to become temporarily deformable into amoeba-like shapes, the surgical opening for introducing the novel IOL into the empty chamber by the novel techniques illustrated previously can be of the order of 1 to 2 mm (and greater if required). In principle, the IOL can be inserted through the pupillary aperture in a partially or essentially dry state (since the lens dimensions are measurably smaller in such state). Such lens assumes its final dimensions after several hours in the eye by swelling to equilibrium in the surrounding vitrium.

In the event the IOL will not maintain its correct position in the human eye, the IOL can be provided with a thread-like mounting means such as textile fibrous material, e.g., Dacron and/or absorbable surgical material, advantageously in a ring-like arrangement about the IOL circumference. Such means should provide maximum non-interference with the optical zone of the intraocular lens. The novel casting polymerization technique can take place in the presence of, for instance, a preshaped reinforcing (e.g., thread-like or filament-shaped) structure, crimped if desired, to provide stretch and elongation characteristics, contained within the concave mold cavity proximate to the shaped edge of the mold perimeter. In its preferred form the resulting IOL lens product possesses an optical zone essentially free from interference of the mounting means thus imbedded therein. Such mounting means can be anchored within the eye, e.g., iris, by known surgical procedures. In one embodiment the mounting means may comprise thread-like or fine knit-like material which extends outwardly from the lens body (preferably proximal to toroidal surface 3). Such material, textile and/or absorbable suture, engage eye tissue proximal thereto, and with the passage of time an intermingling of tissue growth with the said material takes place. The non-absorbable thread-like material will be permanently engaged with the tissue growth; the absorbable thread-like material is, as expected, absorbed into the tissue body. The anchoring technique of the intraocular lens in the above manner is effective and permanent with minimum trauma to the eye. However, as stated previously, it may not be necessary to secure the IOL implant by surgical means. If the IOL is properly chosen in shape and size and is properly inserted into the posterior chamber, especially in those cases where the rear surface and the capsula lentis is not destroyed, the prognosis of a permanent IOL implant is favorable.

Examples 1—4

A solution is prepared using 10 ml of 2-hydroxyethyl methacrylate, 0.03 ml of ethylene glycol dimethacrylate, 0.16 g of sodium methacrylate, and 10 ml of glycerol. To this solution there is added 0.015 g of ammonium peroxodisulfate in the form of a 25% aqueous solution as a polymerization catalyst. The resulting clear liquid is charged into the concave cavity of four separate polypropylene molds shown in Figure 1, the cavity of which is formed from a central spherical cap of a sphere radius 6 mm and width 9 mm continuously linked to a toroidal surface. The toroidal surface is formed by the rotation of a meridian circle of diameter 1 mm, the center of which is distanced from the axis 3.7 mm. The toroidal surface is terminated by a sharp circular edge of diameter 9.4 mm which delimits the sagittal depth of mold equal to 2.9 mm. The cavity volume of this mold is 134 microliters.

The volume of clear polymerizable liquid separately dosed into each of the four molds was 181, 214, 247, and 278 microliters, respectively. The resulting convex menisci surfaces of the dosed molds, for convenience, are shown as 11, 12, 13 and 14, of Figure 1. The molds are maintained in a horizontal position at all times and are carefully inserted into a tunnel heated to 75°C with an inert atmosphere maintained by a moderate stream of nitrogen. Practically complete conversion is achieved after 30 minutes of polymerization. The molds with gel moldings are immersed in distilled water at ambient temperature for 24 hours. The lenses are then released from the molds. To obtain perfect washing, the molded lenses are heated for several days to 80°C in 50% aqueous isopropyl alcohol (fresh aqueous isopropyl alcohol is used daily). Similar washings are carried out with distilled water for several days and the washed lenses are eventually stored in an aqueous solution containing 0.6% NaCl and 0.43% $NaHCO_3$ in a form ready for surgical application. The intraocular lenses made from the above stated four various doses of liquid mixture have the characteristics set out in Table I below:

TABLE I

| IOL | Thickness (mm) | Central curvature (mm)[a] | Refraction (diopters)[b] | Refractive index | Diameter (mm) |
|---|---|---|---|---|---|
| 1 | 4.1 | 15 | 13 | 1.41 | 9.7 |
| 2 | 4.9 | 10 | 15 | 1.41 | 9.7 |
| 3 | 5.6 | 8 | 16.5 | 1.41 | 9.7 |
| 4 | 6.3 | 7 | 18 | 1.41 | 9.7 |

[a] Central curvature of front surface.
[b] Refraction in immersion of physiologic saline.

Figure 1 shows the axial sectional view in which the front surface of the four intraocular lenses corresponds to the curves defined by 11, 12, 13, and 14.

If the polymerizable liquid mixture is prepared by diluting the above stated amount of monomers with a volume of glycerol other than 10 ml, there is obtained an intraocular lens having the same shape but of different size. The size differs from the original IOL size by a factor $f=(0.4+0.04V)^{-1/3}$, wherein $V$ is the amount of added glycerol in ml. Thus, by diluting the monomer mixture with only 7 ml of glycerol, dimensions, i.e., diameter, thickness and radii of curvature, will be increased by the factor 1.14. On the other hand, by diluting with 20 ml of glycerol, the IOL lenses will be contracted by the factor 0.94. Because dilution of the monomer mixture does not substantially affect the resulting refractive index of gel, the refraction is changed so, that the above given values should be divided by the pertinent factor f.

Other lightly crosslinked hydrophilic polymers, desirably those which have a refractive index between about 1.38 and 1.44 in equilibrium with the physiologic medium, can be used similarly as the aforesaid type of gel.

**Claims**

1. A process for fabricating an intraocular lens of predetermined dimensions from a polymerizable hydrogel liquid mixture comprising reactants, characterized by:
    a. introducing the liquid mixture into a cavity (4) of an open mold (10) which has a concave surface (7) corresponding to the convex shape of one surface (2) of the predetermined intraocular lens, said cavity perimeter being delimited by a sharp edge (5);
    b. said liquid mixture being introduced in an amount at least sufficient to approximately reach the level of the sharp edge (5) such that the amount may exceed the volume of the cavity defined by the sharp edge (5), may be level with the sharp edge (5) or may be slightly below the sharp edge (5) in order to form respectively a convex, plano or slightly concave front surface (e.g. 11, 12, 13, 14) which is not in contact with the mold;
    c. polymerizing the liquid mixture without rotating the mold, for a period of time sufficient to form a hydrogel intraocular lens having at least one convex surface;
    d. equilibrating said intraocular lens product by repeating washings in an aqueous medium; and
    e. thereafter storing and maintaining the resulting predetermined intraocular lens, at least one optically finished convex surface of the lens being in osmotic equilibrium with a physiological solution.

2. The process of claim 1 wherein said liquid mixture comprises polymerizable ethylenically unsaturated compound.

3. The process of claim 2 wherein said liquid mixture comprises 2-hydroxyethyl methacrylate.

4. The process of claim 2 wherein a preshaped reinforcing structure is contained within the mold cavity (4) proximal to the sharp edge (5) perimeter thereof, and wherein the resulting intraocular lens hydrogel contains said reinforcing structure proximal to the optical surfaces but essentially free from interference with the optical zone of the said lens.

5. The process of claim 1 wherein the storing and maintaining of the resulting intraocular lens in physiologic solution is provided in flexible sterilized, sealed container which is substantially tubular in shape, one closed end thereof being defined by a narrow diameter which is less than the thickness of the intraocular lens.

# EP 0 166 051 B1

## Patentansprüche

1. Verfahren zur Herstellung einer Intraokularlinse mit vorbestimmten Abmessungen aus einem flüssigen Gemisch eines polymerisierbaren Hydrogels und anderen reaktiven Bestandteilen, gekennzeichnet durch

a. Einbringen des flüssigen Gemisches in einen Hohlraum (4) einer offenen Form (10) mit einer konkaven Oberfläche (7), die der konvexen Form einer Oberfläche (2) der vorbestimmten Intraokularlinse entspricht, wobei der Hohlraumumfang durch eine scharfe Kante (5) begrenzt ist,

b. Einbringen des flüssigen Gemisches in einer solchen Menge, daß das Niveau der scharfen Kante (5) etwa erreicht wird, wobei diese Menge größer als das Volumen des durch die scharfe Kante (5) begrenzten Hohlraums, auf der gleichen Höhe mit oder etwas unterhalb der scharfen Kante (5) sein kann, zur Bildung einer konvexen, planen bzw. schwach konkaven Vorderseite (z.B. 11, 12, 13, 14), die mit der Form nicht in Berührung ist,

c. Polymerisieren des flüssigen Gemsiches oder Rotieren der Form bis zur Bildung einer intraokularen Hydrogellinse mit mindestens einer konvexen Oberfläche,

d. Equilibrieren des intraokularen Linsenprodukts durch wiederholtes Waschen in einem wässrigen Medium und

e. Aufbewahren der entstandenen vorbestimmten Intraokularlinse, wobei mindestens eine optisch fertige konvexe Fläche der Linse mit einer physiologischen Lösung im osmotischen Gleichgewicht ist.

2. Verfahren nach Anspruch 1, gekennzeichnet durch Verwendung eines eine polymerisierbare ethylenisch ungesättigte Verbindung enthaltenden flüssigen Gemischs.

3. Verfahren nach Anspruch 2, gekennzeichnet durch Verwendung eines 2-Hydroxyethylmethacrylat enthaltenden flüssigen Gemisches.

4. Verfahren nach Anspruch 2, dadurch gekennzeichnet, daß in den Hohlraum (4) der Form in der Nähe des Umfangs der scharfen Kante (5) eine Verstärkungsstruktur eingebracht wird und die entstandene intraokulare Hydrogellinse die Verstärkungsstruktur in der Nähe der optischen Flächen enthält, jedoch im wesentlichen frei von Interferenz mit der optischen Zone der Linse ist.

5. Verfahren nach Anspruch 1, dadurch gekennzeichnet, daß das Aufbewahren der entstandenen intraokularen Linse in der physiologischen Lösung in einem flexiblen, sterilisierten, verschlossenen, im wesentlichen rohrförmigen Behälter erfolgt, dessen ein Ende einen Druchmesser aufweist, der geringer als die Dicke der intraokularen Linse ist.

## Revendications

1. Procédé pour la fabrication de lentille intraoculaire de dimensions déterminées à partir d'un mélange liquide d'hydrogel polymérisable comportant des réactants caractérisé en ce que:

a) on introduit le mélange liquide dans l'empreinte (d) d'un moule ouvert (10) qui présente une surface concave (7) correspondante à la forme convexe d'une des surfaces (2) de la lentille intra-oculaire déterminée, le pourtour la ladite empreinte étant délimité par une arête vive (5);

b) on introduit ledit mélange liquide en quantité au moins suffisante pour atteindre approximativement le niveau de l'arête vive (5), de façon que ladite quantité puisse excéder le volume de l'empreinte délimitée par l'arête vive (5), puisse être au ras de l'arête vive (5) ou puisse être légèrement au-dessous de l'arête vive (5) afin de former respectivement une surface avant convexe, plane ou légèrement concave (par exemple 11, 12, 13, 14) qui n'est pas en contact avec le moule;

c) on polymérise le mélange liquide sans faire tourner le moule, pendant un laps de temps suffisant pour façonner une lentille intra-oculaire en hydrogel présentant au moins une surface convexe;

d) on équilibre ladite lentille intra-oculaire ainsi produite par des lavages répétés en milieu aqueux; et

e) on conserve et l'on maintient ensuite le lentille intra-oculaire déterminée résultante, au moins une surface convexe à fini optique de la lentille étant en équilibre osmotique avec une solution physiologique.

2. Procédé selon la revendication 1, dans lequel ledit mélange liquide comprend un composé à insaturation éthylénique polymérisable.

3. Procédé selon la revendication 2 dans lequel ledit mélange liquide comporte le 2-hydroxyéthyl méthacrylate.

4. Procédé selon la revendication 2 dans lequel une structure de renforcement préfaçonnée est contenue à l'intérieur de l'emprIente (4) près du pourtour de son arête vive (5), et dans lequel l'hydrogel de la lentille intra-oculaire résultante contient ladite structure de renforcement près des surfaces optiques mais pratiquement sans qu'elle empiète sur la zone optique de ladite lentille.

5. Procédé selon la revendication 1 dans lequel la conservation de la lentille intra-oculaire résultante et son maintien dans de la solution physiologique sont assurés dans un récipient scellé souple, stérilisé, de forme sensiblement tubulaire, dont une extrémité présente un diamètre faible inférieur à l'épaisseur de la lentille intra-oculaire.

FIG.1

FIG.2

1